(19) Europäisches Patentamt / European Patent Office / Office européen des brevets

(11) **EP 3 118 671 B1**

(12) **EUROPEAN PATENT SPECIFICATION**

(45) Date of publication and mention
of the grant of the patent:
**23.03.2022 Bulletin 2022/12**

(21) Application number: **15761883.6**

(22) Date of filing: **28.01.2015**

(51) International Patent Classification (IPC):
**G02C 7/02** *(2006.01)*     **A61F 2/16** *(2006.01)*
**G02B 7/06** *(2021.01)*     **G02C 7/04** *(2006.01)*
**G02B 3/06** *(2006.01)*

(52) Cooperative Patent Classification (CPC):
**G02C 7/028; A61F 2/1645; G02C 7/02; G02C 7/04;**
A61F 2230/0006; G02B 3/06; G02C 2202/22

(86) International application number:
**PCT/JP2015/052409**

(87) International publication number:
**WO 2015/136997 (17.09.2015 Gazette 2015/37)**

(54) **OPHTHALMIC LENS AND METHOD FOR DESIGNING OPHTHALMIC LENS**

OPHTHALMISCHE LINSEN UND VERFAHREN ZUM ENTWURF OPHTHALMISCHER LINSEN

LENTILLE OPHTALMIQUE ET PROCÉDÉ DE CONCEPTION DE LENTILLE OPHTALMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2014 JP 2014048026**

(43) Date of publication of application:
**18.01.2017 Bulletin 2017/03**

(73) Proprietor: **Kowa Company Ltd.
Naka-ku
Nagoya-shi
Aichi 460-8625 (JP)**

(72) Inventor: **ISHIKAWA, Haruo
Nagoya-shi
Aichi 457-0841 (JP)**

(74) Representative: **Ribeiro, James Michael
Withers & Rogers LLP
2 London Bridge
London SE1 9RA (GB)**

(56) References cited:
WO-A1-01/89424        WO-A1-02/088828
WO-A1-2010/064278     DE-A1-102008 003 770
JP-A- 2002 040 373    JP-A- 2002 122 823
JP-A- 2004 133 024    JP-A- 2008 221 720
JP-A- 2009 535 186    US-A1- 2011 205 486

## Description

[Field]

The embodiments discussed herein pertain to an ophthalmic lens for correcting astigmatism and a method for designing an ophthalmic lens.

[Background]

As examples of an ophthalmic lens for correcting astigmatism, eyeglasses, contact lenses, intraocular lenses and the like are named. In these ophthalmic lenses, a lens surface may have an aspherical shape or an optical surface referred to as a toric surface. It is noted that is a surface shape of a lens where radii of curvature of at least two meridians differ from each other as in the case of a side surface of a rugby ball or a doughnut. Conventionally, ophthalmic lenses for correcting astigmatism have been designed and manufactured using a formula which defines only a lens cross-sectional shape in a lens axis direction and a principal meridian direction, a formula which defines a lens cross-sectional shape based on a distance from an optical axis of a lens and an angle made by a principal meridian and a meridian or the like (patent literature 1 and patent literature 2).

[Citation List]

[Patent Literature]

[PTL 1] Japanese Patent No. 4945558
[PTL 2] JP-A-2011-519682
US-A-2011/205486 relates to a toric optic for ophthalmic use for modifying or reducing non-axisymmetric higher order aberrations.
WO-A-01/89424 relates to a method of obtaining ophthalmic lenses providing the eye with reduced aberrations.
DE-A-10 2008 003 770 relates to safety spectacles for correction of refractive error.

[Summary]

[Technical Problem]

However, in the conventional method for designing an ophthalmic lens for correcting astigmatism which defines only a lens cross-sectional shape in a principal meridian direction, a cross-sectional shape of the entire lens cannot be defined. Further, it is also difficult to define a shape in directions other than the direction adopted in defining a lens cross-sectional shape. Still further, even when an angle such as an angle made by a principal meridian and a meridian is used as a variable, the variable is a value which is uniquely determined once a shape in a steep meridian direction and a flat meridian direction on a plane perpendicular to an optical axis of a lens is determined. As a result, the degree of freedom in designing an ophthalmic lens is limited. Accordingly, even when a lens is manufactured using these designing methods, there is a possibility that aberration cannot be properly corrected over the entire lens.

Recently, along with the development of an aberration measuring device, there has been a demand for a lens where aberration of high order can be also favorably controlled besides aberration of low order such as myopia, hyperopia or astigmatism. Particularly, an aspherical ophthalmic lens has been served for a practical use, and there has been a demand for a method for designing a lens where spherical aberration of the lens can be controlled more favorably. Although this spherical aberration is rotation symmetrical aberration, it is considered that substantially the same phenomenon occurs also with respect to a toric lens.

That is, it is considered that, in a lens, a light beam which passes through a lens at a position away from an optical axis intersects with the optical axis at a position different from a paraxial focal point in a direction of a steep meridian and in a direction of a flat meridian. Accordingly, in a toric lens, it is not always the case where cylindrical refractivity on a para-axis and cylindrical refractivity at a portion away from the optical axis agree with each other. Rather, it is natural to consider that the cylindrical refractivity does not agree with each other. However, in the prior art, there has been proposed no method for designing a lens which uses a formula capable of advantageously controlling the cylindrical refractivity in a paraxial region and in a non-paraxial region while defining a toric surface over the entire lens.

The technique of this disclosure is made in view of the above-mentioned circumstances, and it is an object of this disclosure to realize an ophthalmic lens and a method for designing an ophthalmic lens where a toric surface and a spherical/aspherical shape and the like can be defined over the entire lens surface.

[Solution to Problem]

An ophthalmic lens according to the present disclosure is in accordance with claim 1 and a method for designing the ophthalmic lens of claim 1 is defined in claim 3. With such a configuration, it is possible to design the ophthalmic lens while controlling cylindrical refractivity over the entire lens surface in the arbitrary direction from the lens center. That is, a cross sectional shape in the arbitrary direction is expressed by a general formula on an aspherical surface. Therefore, paraxial refractivity and aberration can be calculated, particularly spherical aberration can be obtained easily and strictly within a cross section in such a direction.

Further, coefficients of $r^n$ in second and subsequent terms in the formula (1) are functions having a period of 180° with respect to an angle about the optical axis. In addition, A ($\theta$) in the formula (1) is a function having a period of 180°, and B($\theta$) is a function having a period of 180° or a sum of the function having a period of 180° and the function having a period of 90°.

[Advantageous Effects of Invention]

According to the technique of this disclosure, it is possible to realize an ophthalmic lens and a method for designing the ophthalmic lens which can define a toric surface and a spherical/aspherical surface and the like over the entire lens surface. Further, it is possible to effectively reduce or control aberration of high degrees (particularly, spherical aberration and tetra foil aberration).

[Brief Description of Drawings]

FIG. 1 is a schematic diagram illustrating spherical aberration of a lens;

FIG. 2A and FIG. 2B are schematic diagrams illustrating spherical aberration of a toric lens;

FIG. 3 is a schematic diagram illustrating a method of calculating spherical aberration of the toric lens;

FIG. 4 is a diagram illustrating one example of a simulation result of a toric intraocular lens according to one embodiment and a conventional toric intraocular lens;

FIG. 5 is a diagram illustrating the schematic configuration of a schematic eye for evaluating the toric intraocular lens according to one embodiment;

FIG. 6 is a table illustrating one example of an evaluation result of the schematic eye illustrated in FIG. 4;

FIGS. 7A to 7C are graphs illustrating one example of an MTF measurement result of the toric intraocular lens according to one embodiment;

FIGS. 8A to 8C are graphs illustrating one example of an MTF measurement result of the conventional toric intraocular lens;

FIG. 9 is a graph illustrating a change in the amount of sag of a toric surface of the toric intraocular lens according to one embodiment.

FIG. 10 is a table illustrating one example of an evaluation result with respect to axis misalignment of the toric lens according to one embodiment;

FIG. 11 is a table illustrating one example of a result of optical simulation with respect to axis misalignment of the toric lens according to one embodiment;

FIG. 12A is a table illustrating one example of a result of optical simulation with respect to axis misalignment of the toric lens according to one embodiment; and

FIG. 12B is a table illustrating one example of a result of optical simulation with respect to axis misalignment of the toric lens according to one embodiment.

[Description of Embodiments]

Hereinafter, the mode for carrying out the present invention is described. Although a toric intraocular lens will be described in the description made hereinafter, the present invention is not limited to the intraocular lens and is also applicable to various ophthalmic lenses including contact lenses.

Firstly, formulas used in designing a conventional toric intraocular lens are described with reference to the above-mentioned patent documents. In the toric intraocular lens, depending on a toric surface, refractivity of a lens differs between directions (a first meridian direction and a second meridian direction) which are orthogonal to each other and set on the toric surface. Astigmatism can be corrected by making use of this difference in refractivity. In general, this difference in refractivity is referred to as cylindrical refractivity. On a toric surface, a meridian in a direction that refractivity is large is referred to as a steep meridian, and a meridian in a direction that refractivity is small is referred to as a flat meridian. Further, an average value of refractivity on the two meridians is referred to as equivalent spherical power (or simply referred to as spherical power). Usually, in an ophthalmic lens for correcting astigmatism, as indexes indicative of optical performances, equivalent spherical power and cylindrical refractivity are used.

In the description made hereinafter, a steep meridian direction of the toric intraocular lens is assumed as an X direction and a flat meridian direction of the toric intraocular lens is assumed as a Y direction. However, it is self-explanatory that the X direction and the Y direction can be reversed. The detail of the methods of deriving formulas described hereinafter is described in various patent documents and hence, the detail of the methods of deriving the formulas is omitted in this specification. As a formula for defining a conventional toric surface, a formula (7) which expresses a lens cross-sectional shape taken along a plane including an X axis and an optical axis, and a formula (8) which expresses a lens cross-sectional shape taken along a plane including a Y axis and the optical axis can be named. In these formulas, Rx and Ry are respectively a radius of curvature in cross section of the lens taken along a plane including the X axis and the optical axis and a radius of curvature in cross section of the lens taken along a plane including the Y axis and the optical axis. In these formulas, Rx and Ry are not equal (Rx≠Ry). cx and cy are respectively a curvature in cross section of the lens taken along a plane including the X axis and the optical axis and a curvature in cross section of the lens taken along a plane including the Y axis and the optical axis. Here, cx is 1/Rx (cx=1/Rx), and cy is 1/Ry (cy=1/Ry). kx and ky are respectively a conic constant in the X direction and a conic constant in the Y direction. In Japanese

Patent No. 4945558, there is a description that kx and ky are not equal (kx≠ky).

$$Z_x = \sqrt{2R_x x - (1+k_x)x^2}$$

$$(7)$$

$$Z_y = \sqrt{2R_y y - (1+k_y)y^2}$$

$$(8)$$

Further, as a formula used for designing a conventional toric intraocular lens, formulas (9) and (10) can be named in place of the formulas (7) and (8). In the formulas (9) and (10), Rx and Ry are not equal (Rx≠Ry). In Japanese Patent No. 4945558, there is a description that kx and ky are not equal (kx≠ky).

$$Z_x = \frac{x^2 / R_x}{1 + \sqrt{1 - (1+k_x)x^2 / R_x^2}} + \sum_j c_j x^j$$

$$(9)$$

$$Z_y = \frac{y^2 / R_y}{1 + \sqrt{1 - (1+k_y)y^2 / R_y^2}} + \sum_j c_j y^j$$

$$(10)$$

When the formulas (7) and (8) or the formulas (9) and (10) are used, only a lens cross-sectional shape in the X direction and a lens cross-sectional shape in the Y direction can be defined, and a cross-sectional shape of the entire lens cannot be defined.

Also known is a method where a toric intraocular lens is designed using a formula (11).

$$Z(r,\theta) = \frac{(c_x \cos^2 \theta + c_y \sin^2 \theta)r^2}{1 + \sqrt{1 - (1+k_x)c_x^2 r^2 \cos^2 \theta - (1+k_y)c_y^2 r^2 \sin^2 \theta}}$$

$$(11)$$

However, even when the formula (11) is used, when shapes of the lens in the X direction and the Y direction are determined, that is, when cx, cy, kx, and ky are determined, shapes of the lens in directions other than two directions are also uniquely determined and hence, the degree of freedom in defining a cross-sectional shape of the entire lens is small.

In view of the above, in the present invention, an intraocular lens is manufactured by defining a lens surface using the following formula (12). A first term of the formula (12) defines a lens surface which is in rotation symmetry with respect to an optical axis of the lens, and second and succeeding terms define a toric surface.

$$Z = \frac{cr^2}{1+[1-c^2r^2(k+1)]^{\frac{1}{2}}} + a_{2x}X^2 + a_{2y}Y^2 + a_{4x}X^4 + a_{2x2y}X^2Y^2 + a_{4y}Y^4 + \cdots$$

$$(12)$$

In the formula, c is a curvature of a reference surface which is in rotation symmetry with respect to the optical axis of the lens before a toric surface defined by the second and succeeding terms of the formula (12) is added. X and Y are distances from the center of the lens in the first direction and the second direction respectively. For example, X and Y are distances from the center of the lens in the steep meridian direction and in the flat meridian direction. r is a distance ($r^2=X^2+Y^2$) in the radial direction. k is a conic constant of the reference surface which is in rotation symmetry with respect to the optical axis of the lens before a toric surface defined by the second and succeeding terms of the formula (12) is added. c, r and k are used in common in the X direction and in the Y direction. Further, a is a parameter used in adding a toric surface . The second and succeeding terms of the formula (12) are respective terms obtained by developing $(X^2+Y^2)^n$ (n=1, 2 ...). Coefficients in respective terms of second and succeeding terms are parameters used in adding a toric surface. The first term of the formula (12) is one example of a predetermined definition formula for defining a lens surface which is in rotation symmetry with respect to the optical axis of the lens. The first term of the formula (12) can be replaced with other formulas when the formulas define a lens surface substantially in the same manner as the first term does.

With the use of the above-mentioned formula, a lens surface can be defined over the entire lens. Accordingly, the lens surface can be defined with higher degree of freedom in designing a cross-sectional shape of a lens compared to the prior art. Particularly, a shape in the direction other than the X direction and the Y direction (for example, the direction that X and Y are equal (X=Y)) which has not been defined by the conventional formulas as described above can be also freely defined.

The first term of the formula (12) has the same form as a formula on a spherical lens or a formula on an aspherical lens which includes only a conic constant. Accordingly, in designing a toric intraocular lens using the formula (12), a base shape of the toric intraocular lens can be formed into a rotation symmetrical lens substantially in the same manner as the prior art. Accordingly, the toric intraocular lens which is designed using the formula (12) and is manufactured can be installed into a conventional insertion instrument without any problems.

Conventionally, there has been proposed a method for equating an edge thickness of a lens in a direction of 45° with that of a rotation symmetrical lens (for example, patent literature 2 and the like). However, an edge thickness cannot be calculated until parameters of the toric surface are determined. On the other hand, in the designing method of the present invention which uses the formula (12), by setting a coefficient of $X^{2j}Y^{2(n-j)}$ (j: natural number other than n) to 0 or by setting $a_{2qx}$, $-a_{2qy}$, and $a_{2qx}a_{2py}$ as $a_{2qx}=-a_{2qy}$ and $a_{2qx}a_{2py}=0$ (p, q: natural numbers), it is unnecessary to calculate an edge thickness and hence, it is possible to design a shape of a lens having substantially the same edge thickness in a direction of 45° as a rotation symmetrical lens.

In manufacturing a lens by a so-called molding method, it is necessary to take into account a change in lens shape caused by shrinkage of a lens material. In designing a lens using the formula (12) of the present invention, when a base shape of the lens is equal to that of a rotation symmetrical lens, it can be regarded that a shrinkage rate of the lens is substantially equal to that of the rotation symmetrical lens. Accordingly, with the use of the lens designing method of the present invention, it is possible to evaluate a shrinkage rate more efficiently than the conventional method where a shrinkage rate is evaluated based on a toric intraocular lens which is a rotation asymmetrical lens.

As described later, paraxial curvatures in the X direction and in the Y direction can be also easily calculated. Therefore, paraxial refractivity can be also easily calculated. Accordingly, the paraxial powers can be easily calculated based on the function of the formula (12). Further, with the use of the formula (12), it is possible to control spherical aberrations of the toric intraocular lens in the X direction and in the Y direction. In this manner, by designing the lens using the formula (12), the degree of freedom of parameters which define a toric surface of the toric intraocular lens is increased so that it is possible to design a lens surface shape which corrects various aberrations more suitably than the prior art.

Hereinafter, embodiments relating to an intraocular lens and a method for designing an intraocular lens using the above-mentioned formula (12) are described.

(Embodiment 1)

Embodiment 1, not part of the invention, is described hereinafter. With respect to an intraocular lens, according to paraxial optics, paraxial refractivity P(D) is expressed by the following formula (13).

$$P = (n_e - n_e^{medium}) \cdot \left[ \left( \frac{1}{R1} \right) - \left( \frac{1}{R2} \right) \right] + \frac{(n_e - n_e^{medium})^2 \cdot t}{n_e \cdot R1 \cdot R2}$$

$$(13)$$

$n_e$ is a refractive index of the intraocular lens on an e line ($\lambda$=546 nm), $n_e^{medium}$ is a refractive index of a medium surrounding the lens on the e line, R1 is a radius of curvature of the lens center on a front surface of the intraocular lens, R2 indicates a radius of curvature of the lens center on a rear surface of the intraocular lens, and t is a thickness of the lens center of the intraocular lens.

When the intraocular lens is a toric intraocular lens, the value of R1 or the value of R2 differs between the X direction and the Y direction. That is, assuming that an R2 surface is formed of a toric surface, power in the X direction and power in the Y direction are respectively expressed by the following formulas (14) and (15).

$$P = (n_e - n_e^{medium}) \cdot \left[ \left( \frac{1}{R1} \right) - \left( \frac{1}{R2_x} \right) \right] + \frac{(n_e - n_e^{medium})^2 \cdot t}{n_e \cdot R1 \cdot R2_x}$$

$$(14)$$

$$P = (n_e - n_e^{medium}) \cdot \left[ \left( \frac{1}{R1} \right) - \left( \frac{1}{R2_y} \right) \right] + \frac{(n_e - n_e^{medium})^2 \cdot t}{n_e \cdot R1 \cdot R2_y}$$

$$(15)$$

Here, the difference between Px and Py is cylindrical refractivity, and (Px+Py)/2 is equivalent spherical power.

Curvatures in the X direction and in the Y direction can be obtained by the formula (12) as follows. Firstly, a curvature c can be expressed by a formula (16) as an inverse number of a radius of curvature R.

$$c = \frac{1}{R}$$

$$(16)$$

Further, a radius of curvature R at a point x in a function f (x) can be expressed by the following formula (17).

$$R = \frac{\left[ 1 + \{f'(x)\}^2 \right]^{\frac{3}{2}}}{f''(x)}$$

$$(17)$$

In general, a function f(x) which expresses a surface of an optical lens can be regarded as a function which passes an origin which is an intersection point between a lens surface and an optical axis of the lens, and is in symmetry with respect to an optical axis. In this embodiment, assume an optical axis as a Z axis. That is, a radius of curvature R can be obtained by substituting 0 for x (x=0) in the formula (17), and a curvature can be also obtained as an inverse number of R.

For example, a curvature c' in an aspherical surface Z(X) expressed by the following formula (18) is obtained.

$$Z(X) = \frac{cX^2}{1+[1-c^2X^2(k+1)]^{\frac{1}{2}}} + a_{2x}X^2 + a_{4x}X^4 + a_{6x}X^6 + \cdots$$

$$(18)$$

Firstly, with respect to a first derivative value Z'(X) of Z(X), even when the first derivative is performed, a variable X remains in all terms. Accordingly, when 0 is substituted for X (X=0) in Z'(X), a value of Z'(0) becomes 0 (Z'(0)=0).

Next, a second derivative value Z"(X) of Z(X) is given by a formula (19).

$$Z''(X) = \frac{c}{2}[1-c^2x^2(k+1)]^{-\frac{3}{2}} \cdot \left\{-2c^2x(k+1)\right\} \cdot x + c[1-c^2x^2(k+1)]^{\frac{1}{2}} + 2a_{2x} + 12a_{4x}x^2 + \cdots$$

$$(19)$$

Here, when 0 is substituted for X (X=0) in Z" (X), the following formula (20) can be obtained.

$$Z''(0) = c + 2a_{2x}$$

$$(20)$$

From the above, the curvature c' is expressed by the following formula (21).

$$c' = c + 2a_{2x}$$

$$(21)$$

In the same manner as described above, in the formula (12) which expresses a lens surface of the present invention, curvatures cx and cy in the X direction and in the Y direction are respectively expressed by the following formulas (22) and (23).

$$c_x = c + 2a_{2x}$$

$$(22)$$

$$c_y = c + 2a_{2y}$$

$$(23)$$

In this manner, cx and cy can be directly calculated from $a_{2x}$, $a_{2y}$ and c and hence, Rx and Ry can be also calculated. Accordingly, by using the formula (12), it is possible to design and provide a toric intraocular lens by arbitrarily specifying refractivity (paraxial) in the X direction and refractivity (paraxial) in the Y direction.

(Embodiment 2)

Next, the embodiment 2, not part of the invention, is described. In the formula (12) of the present invention, when all parameters in the second and succeeding terms are set to 0, a formula which expresses a rotation symmetrical lens can be obtained by the first term. That is, the formula (12) of the present invention defines a toric surface having a curvature c, a conic constant k, and a rotation symmetrical surface as a base shape. Therefore, assuming that $a_{2qx}$ and $-a_{2qy}$ are equal to each other ($a_{2qx}=-a_{2qy}$) and $a_{2qx2py}$ is 0 ($a_{2qx2py}=0$) (p, q: natural numbers), equivalent spherical power which is an average of a steep meridian and a flat meridian of the toric intraocular lens can be defined as refractivity generated by a rotation symmetrical surface having a curvature c and a conic constant k. That is, a paraxial curvature which defines equivalent spherical power of a toric surface can be easily obtained from the first term of the formula (12). As a result, equivalent spherical refractivity can be also easily calculated. In this manner, in the formula (12), by merely setting two parameters, that is, a radius of curvature R (c=1/R) of a reference surface shape and a pair of coefficients ($a_{2qx}$=-$a_{2qy}$) having the same absolute value and being opposite to each other only in positive/negative signs, it is possible to design a toric intraocular lens by specifying equivalent spherical power and cylindrical refractivity.

Refractivity of the steep meridian and refractivity of the flat meridian are refractivity allocated from equivalent spherical power, and the refractivity can be calculated by using curvatures which are expressed by the following formulas (24) and (25) .

$$c_x = c + 2a_{2x}$$

$$(24)$$

$$c_y = c - 2a_{2x}$$

$$(25)$$

The description has been made above with respect to the case where the toric intraocular lens is designed by merely setting parameters thus requiring no complicated calculation. However, even when $a_{2qx}$ is not equal to -$a_{2qy}$ ($a_{2qx} \neq -a_{2qy}$) and $a_{2qx2py}$ is not 0 ($a_{2qx2py} \neq 0$) in the formula (12) of the present invention, it is also possible to calculate a curvature which defines equivalent spherical power by performing simple calculation where $a_{2x}$ and $a_{2y}$ are added to a curvature c of a rotation symmetrical surface.

As described above, the fact that equivalent spherical refractivity of a toric intraocular lens can be calculated using the formula (12) is advantageous in designing a lens which is required in a cataract operation. The reason is as follows. Firstly, a toric intraocular lens is inserted into an eyeball of a patient for reducing astigmatism. In general, a result of a visual function test carried out in an ophthalmic clinic is outputted in the form of spherical power and astigmatic power. For example, when a test result is "S+5.00C-1.00A×90°", this test result indicates that spherical power (equivalent spherical power) is +5.00 D, astigmatic power is -1.00 D, and astigmatic axis is 90°. Accordingly, to take into account that a result of a visual function test is given in the form of spherical power and astigmatic power (astigmatic axis angle), the designing of a toric intraocular lens by the formula (12) using (equivalent) spherical power and astigmatic power as inputs is useful.

(Embodiment 3)

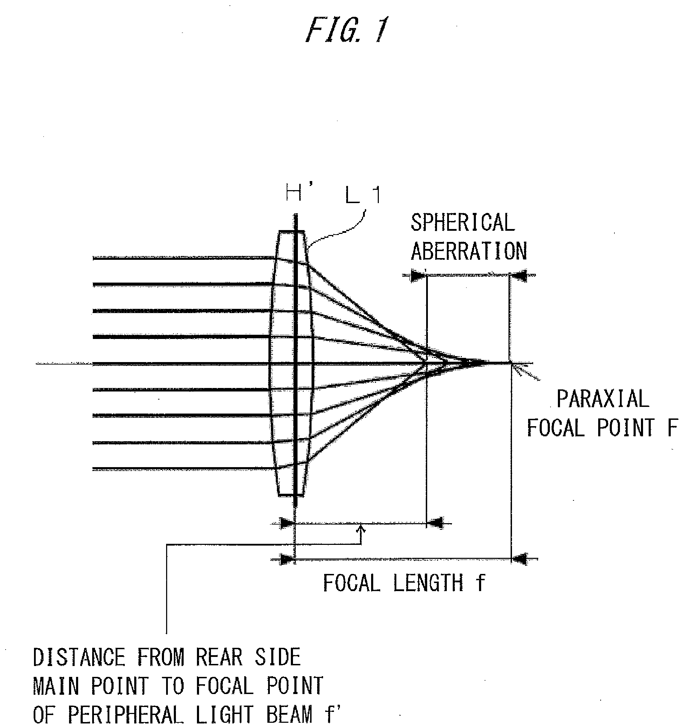

Next, Embodiment 3, not part of the invention, is described. Firstly, spherical aberration which is one of Seidel's five aberrations is described with reference to FIG. 1. When spherical aberration occurs, power difference is generated between a center portion (paraxial portion) and a peripheral portion of a rotation-symmetry-type lens. Spherical aberration is a phenomenon where a light beam which passes through the center portion of the lens and a light beam which passes through the peripheral portion of the lens are not converged to the same focal point.

As illustrated in FIG. 1, assuming a rear principal point of a lens L1 as H' , paraxial focal point as F, a focal distance as f, and a distance from the rear principal point H' to a focal point of a peripheral light beam (a light beam which passes through a peripheral portion of the lens) as f', paraxial refractivity P of the lens L1 on the para-axis is expressed by the following formula (26) using the focal distance f. Although the description is made with respect to the case where air is present in front of and behind the lens and a refractive index is set to 1, the present invention is not limited to such a case.

$$P = \frac{1}{f}$$

$$(26)$$

In this embodiment, the case where spherical aberration occurs means the case where the position of a focal point F' of a peripheral light beam and the position of the paraxial focal point F differ from each other, and paraxial refractivity P' of a peripheral portion of the lens L1 is expressed by the following formula (27) using the distance f'.

$$P' = \frac{1}{f'} \qquad (27)$$

In FIG. 1, the focal point F' of the peripheral light beam is closer to the rear principal point H' than the paraxial focal point F is and hence, the following formula (28) is established. That is, when spherical aberration occurs in the lens L1, difference is generated in refractivity between the center portion and the peripheral portion of the lens L1.

$$P < P' \qquad (28)$$

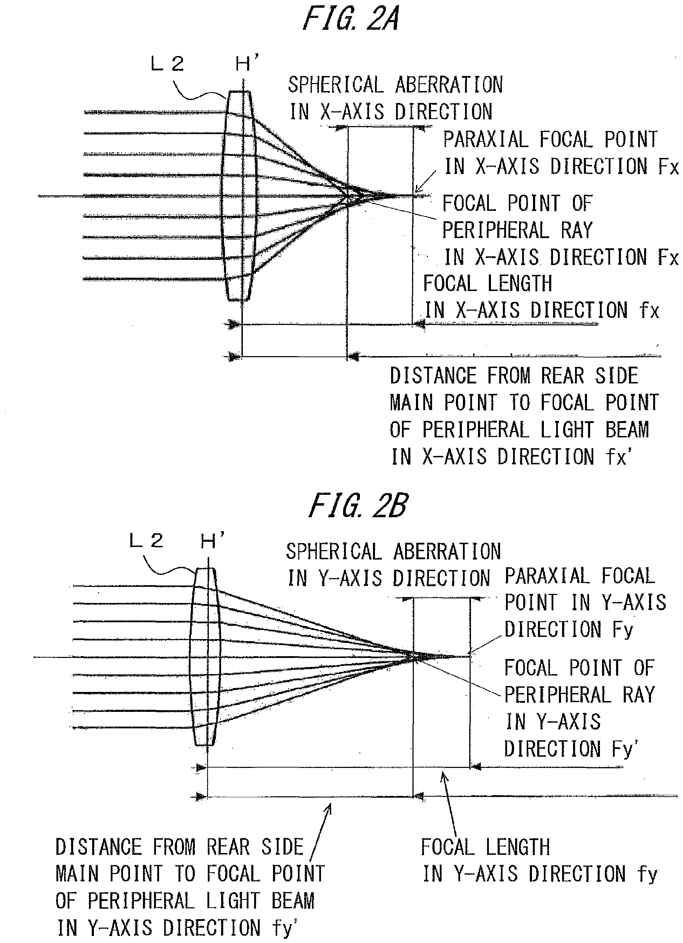

Next, spherical aberration which occurs in a toric lens L2 is described with reference to FIG. 2. For the sake of convenience of description, the description is made assuming that an optical axis of the toric lens L2 is taken on a Z axis, an XY plane orthogonal to the optical axis is set and an X axis and a Y axis are orthogonal to each other. However, the X axis and the Y axis may not be always orthogonal to each other, and may be configured such that the X axis extends in a first direction on the XY plane and the Y axis extends in a second direction which differs from the first direction. FIGS. 2A and 2B illustrate states where light beams in the X direction and Y direction of the toric lens L2 are converged respectively. As illustrated in FIG. 2A, assume a rear principal point of the toric lens L2 as H', a paraxial focal point in the X direction of the lens as Fx, a focal distance in the X direction of the lens as fx, and a distance from the rear principal point H' to a focal point of a peripheral light beam in the X direction of the lens as fx'. In the same manner, as illustrated in FIG. 2B, Fy, fy, fy' are respectively set also with respect to the Y direction of the lens.

In this case, as described with reference to FIG. 1, the following formulas (29) to (32) are established.

$$P_x = \frac{1}{f_x} \qquad (29)$$

$$P_x' = \frac{1}{f_x'} = \frac{1}{f_x + SA_x} \qquad (30)$$

$$P_y = \frac{1}{f_y} \qquad (31)$$

$$P_y' = \frac{1}{f_y'} = \frac{1}{f_y + SA_y} \qquad (32)$$

Here, SAx and SAy indicate a distance of a spherical aberration amount in the X direction of the lens and a distance of a spherical aberration amount in the Y direction of the lens respectively. For example, SAx and SAy in the toric lens L2 illustrated in FIG. 2A and FIG. 2B take negative values.

Cylindrical refractivity Pc of the toric lens L2 in a paraxial portion and cylindrical refractivity Pc' of the toric lens L2 in a peripheral portion are expressed by the following formulas (33) and (34).

$$P_C = P_x - P_y$$

$$(33)$$

$$P_C' = P_x' - P_y'$$

$$(34)$$

In general optical software, spherical aberration is calculated in the Y direction. Accordingly, to calculate spherical aberration in the X direction, it is necessary to rotate lens data by 90° or to exchange parameters of a toric surface. Further, in calculating spherical aberration in the X direction, spherical aberration in the Y direction cannot be calculated. However, when the calculation of spherical aberration in the X direction and the calculation of spherical aberration in the Y direction cannot be simultaneously performed, there is a possibility that operability of designing a toric intraocular lens is lowered. In view of the above, this embodiment proposes a technique by which spherical aberration SA in the X direction is obtained as follows.

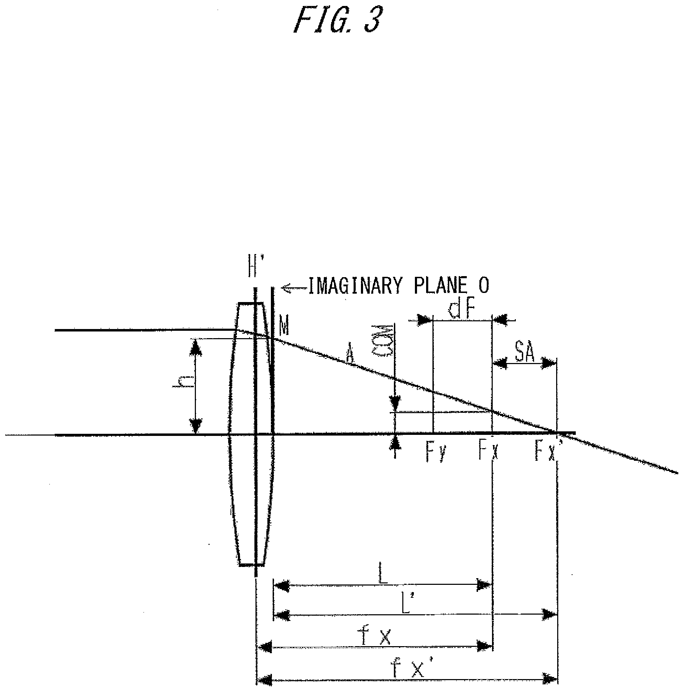

To calculate cylindrical refractivity of a peripheral portion of a lens, respective parameters are defined as illustrated in FIG. 3. In FIG. 3, assume a paraxial focal point in the X direction as Fx, a paraxial focal point in the Y direction as Fy, and the difference between Fx and Fy as dF. Further, assume a paraxial focal point distance in the X direction as fx, and an intersection point between an arbitrary peripheral light beam and an imaginary plane O as M. Still further, assume a height of the intersection point M from an optical axis extending along the imaginary plane O as h, a distance between the paraxial focal point Fx on a paraxial focal point surface and a peripheral light beam A as COM, and a distance between the paraxial focal point Fx and a focal point Fx' of the peripheral light beam A as SA (=spherical aberration). So long as the imaginary plane O is arranged at any position within a range from a rear surface of the lens to a focal point. Any peripheral light beam may be selected as desired provided that a height of light beam is lower than 1/2 of an incident pupil diameter. Further, a raw material for forming a lens and a medium around the lens can be arbitrarily set.

From FIG. 3, the following formula (35) is established.

$$\frac{COM}{SA} = \frac{h}{L + SA}$$

$$(35)$$

From the formula (35), the following formula (36) is obtained.

$$SA = \frac{L \times COM}{h - COM}$$

$$(36)$$

By substituting SAx in the formula (36) for SA in formula (30), it is possible to calculate power in the X direction on a periphery (an arbitrary incident pupil height) of the lens. The paraxial power in the X direction may be obtained from x or may be obtained as a sum of Fy and dF.

Values L, COM, and h in the formulas (35) and (36) can be calculated by general optical software, and it is an easy operation to evaluate such calculation at the time of designing. dF can be also calculated by general optical software. In this modification, it is possible to design a toric intraocular lens by calculating and evaluating both the distribution of refractivity in the X direction and the distribution of refractivity in the Y direction simultaneously with respect to a lens surface which is defined by the formula (12). Here, provided that a peripheral light beam is a light beam which passes through an incident pupil, any peripheral light beam may be set as desired. By calculating refractivity of light beams which are incident on the lens at positions away from an optical axis by fixed distances simultaneously, it is possible to design a toric intraocular lens by calculating and evaluating a change in power.

Next, as one example of this embodiment, with respect to a lens surface defined by the formula (12), a result of evaluation on powers in the X direction and power in the Y direction which use the formulas (29) to (32) is described. Lens data of the lens are illustrated in Table 1 described below. In Table 1, R is a radius of curvature, t is a thickness, n is a refractive index, D is a radius, k is a conic constant, and A is a parameter used for adding a toric surface.

[Table 1]

| No | SURFACE | R(mm) | t(mm) | n(546nm) | D(mm) | k | A |
|---|---|---|---|---|---|---|---|
| 0 | OBJECT SURFACE | inf | inf | 1.336 | 0.00 | - | - |
| 1 | DIAPHRAGM SURFACE | inf | 0.000 | 1.336 | 3.00 (DIAPHRAGM RADIUS) | - | - |
| 2 | R1 SURFACE (SPHERICAL) | 15.7933 | 0.800 | 1.4938 (PMMA) | 3.00 | - | - |
| 3 | R2 SURFACE (TORIC) | -15.6812 | 5.000 | 1.336 | 3.00 | - | See TABLE 2 |
| 4 | IMAGINARY PLANE | inf | 66.830 | 1.336 | 2.77 | - | - |
| 5 | IMAGE PLANE | inf | 0.000 | 1.336 | 0.08 | - | - |

Further, coefficients in the formula (12) are set in accordance with the following Table 2.

[Table 2]

| $a_{2x}$ | $a_{2y}$ | $a_{4x}$ | $a_{2x2y}$ | $a_{4y}$ |
|---|---|---|---|---|
| -4.7786E-03 | 4.7786E-03 | 9.2081E-06 | 0.0000E+00 | -9.2081E-06 |

In this case, power of the lens in the X direction and power of the lens in the Y direction with respect to a pupil diameter are illustrated in the following Table 3.

[Table 3]

| HEIGHT OF PUPIL (mm) | Px(D) | Py(D) | CYLINDRICAL REFRACTIVITY (D) | EQUIVALENT SPHERICAL POWER (D) |
|---|---|---|---|---|
| 0.0 | 21.50 | 18.50 | 3.0 | 20.00 |
| 1.0 | 21.55 | 18.55 | 3.0 | 20.05 |
| 1.5 | 21.62 | 18.62 | 3.0 | 20.12 |
| 2.0 | 21.72 | 18.72 | 3.0 | 20.22 |
| 2.5 | 21.84 | 18.84 | 3.0 | 20.34 |
| 3.0 | 22.00 | 19.00 | 3.0 | 20.50 |

In this case, cylindrical refractivity takes a fixed value regardless of a pupil diameter. Power in the X direction and power in the Y direction change depending on a pupil diameter. That is, these powers are gradually increased from the center to the periphery of the lens at a pitch of 0.5 D. Also equivalent spherical power which is an average of power in the X direction and power in the Y direction is gradually increased from the center to the periphery of the lens at a pitch of 0.5 D.

Next, as another example of this embodiment, a lens having lens data illustrated in the following Table 4 is used.

[Table 4]

| No | SURFACE | R(mm) | t(mm) | n(546nm) | D (mm) | k | A |
|---|---|---|---|---|---|---|---|
| 0 | OBJECT SURFACE | inf | inf | 1.336 | 0.00 | - | - |
| 1 | DIAPHRAGM SURFACE | inf | 0.000 | 1.336 | 3.00 (DIAPHRAGM RADIUS) | - | - |
| 2 | R1 SURFACE (SPHERICAL) | 13.6077 | 0.800 | 1.4938 (PMMA) | 3.00 | - | - |

(continued)

| No | SURFACE | R(mm) | t(mm) | n(546nm) | D (mm) | k | A |
|---|---|---|---|---|---|---|---|
| 3 | R2 SURFACE (TORIC) | -18.6713 | 5.000 | 1.336 | 3.00 | - | See TABLE 5 |
| 4 | IMAGINARY PLANE | inf | 66.768 | 1.336 | 2.79 | - | - |
| 5 | IMAGE PLANE | inf | 0.000 | 1.336 | 0.16 | - | - |

Further, coefficients in the formula (12) are set in accordance with the following Table 5.

[Table 5]

| $a_{2x}$ | $a_{2y}$ | $a_{4x}$ | $a_{2x2y}$ | $a_{4y}$ |
|---|---|---|---|---|
| -4.7998E-03 | 4.7657E-03 | 8.9565E-05 | 0.0000E+00 | 2.5020E-04 |

In this embodiment, power of the lens in the X direction and power of the lens in the Y direction with respect to a pupil diameter are illustrated in the following Table 6.

[Table 6]

| HEIGHT OF PUPIL (mm) | Px(D) | Py(D) | CYLINDRICAL REFRACTIVITY(D) | EQUIVALENT SPHERICAL POWER (D) |
|---|---|---|---|---|
| 0.0 | 21.50 | 18.50 | 3.00 | 20.00 |
| 1.0 | 21.50 | 18.39 | 3.11 | 19.95 |
| 1.5 | 21.50 | 18.25 | 3.25 | 19.88 |
| 2.0 | 21.50 | 18.06 | 3.44 | 19.78 |
| 2.5 | 21.50 | 17.81 | 3.69 | 19.66 |
| 3.0 | 21.50 | 17.50 | 4.00 | 19.50 |

In this case, power in the X direction takes a fixed value regardless of a pupil, that is, 21.5 D. Power in the Y direction is decreased depending on the pupil, and cylindrical refractivity is increased from the center to the periphery of the lens by 1 D. Further, equivalent spherical power is decreased from the center to the periphery of the lens only by 0.5 D which is half of an amount of change in cylindrical refractivity.

As described in the above-mentioned embodiments, according to this embodiment, in designing a lens using the formula (12), it is possible to easily control a change in power in the X direction and in the Y direction of the lens thus designing and providing a toric intraocular lens having various distribution of refractive power.

In ophthalmic clinic industry, the above-mentioned distribution of refractive power is referred to as a power map, and is used for detecting abnormality in shape of cornea of a patient. For example, in an anterior eye part shape analyzer such as Pentacam (registered trademark) (made by OCULUS) or TMS-5 (made by TOMEY CORPORATION), by measuring a shape of cornea, it is possible to grasp how power is distributed in a cornea ranging from the center to the periphery of the cornea. Further, in an intraocular lens inspection apparatus such as IOLA plus (made by ROTLEX), a power map of a lens can be measured. Accordingly, distribution of refractive power in an eyeball of a patient can be obtained by these apparatus. By using the above-mentioned formula (12) based on the obtained distribution of refractive power, it is possible to design and provide an optimum toric intraocular lens.

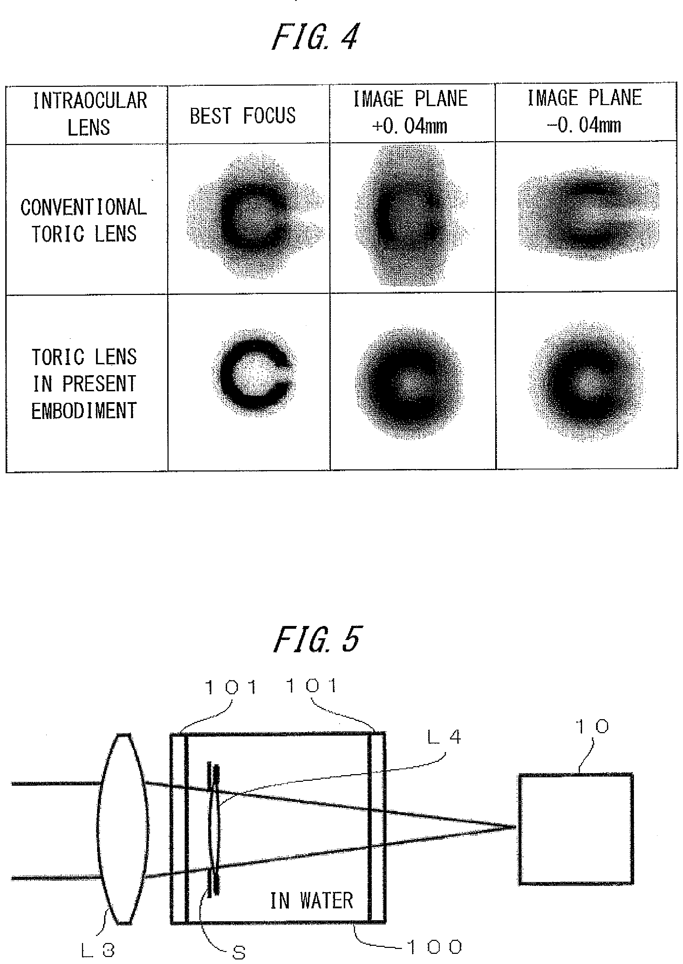

Next, in this embodiment, with reference to FIG. 4, the description is made with respect to a simulation result on Landolt ring images obtained by a toric intraocular lens when the toric intraocular lens is designed using the formula (12). FIG. 4 illustrates Landolt ring images in so-called best focusing where a conventional toric lens and a toric lens according to this embodiment are applied to an astigmatic eye under a predetermined condition as intraocular lenses, Landolt ring images obtained when an image plane is moved away from the lens by 0.04 mm (+) in the simulation, and Landolt ring images obtained when the image plane is made to approach the lens by 0.04 mm (-) in the simulation.

As can be understood from the Landolt rings in best focusing illustrated in FIG. 4, in the toric intraocular lens according to this embodiment, astigmatism is favorably reduced so that a clear Landolt ring image is acquired. On the other hand, in the conventional toric intraocular lens, although a Landolt ring image is confirmed to an extent that the images are recognizable, so-called blurring is generated around the Landolt ring.

Further, the difference in Landolt ring image appears also when an image plane is moved so that the images are defocused. As illustrated in FIG. 4, in the toric intraocular lens according to this embodiment, even when an image plane is displaced from the position for best focusing, blurring having a rotation symmetrical shape is generated. That is, it is safe to say that astigmatism is substantially completely eliminated. On the other hand, in the conventional toric intraocular lens, blurring extends in a longitudinal direction (vertical direction on a paper on which FIG. 4 is drawn) (image plane: +0.04 mm) or extends in a lateral direction (left-and-right direction on the paper on which FIG. 4 is drawn) (image plane: -0.04 mm) and hence, it is understood that astigmatism is not completely eliminated. From the above, it is safe to say that the toric intraocular lens according to this embodiment can acquire more effective astigmatism correction effect compared to the prior art.

Next, the description is made on an evaluation result with respect to the case where an astigmatism cornea lens which differs in astigmatism amount between a center portion and a peripheral portion of the lens is manufactured. FIG. 5 illustrates the schematic configuration of a schematic eye used for the evaluation. In the schematic eye illustrated in FIG. 5, an astigmatism cornea lens L3 is suitably rotatable about an optical axis. Accordingly, it is possible to make an astigmatism axis of the astigmatism cornea lens L3 and an astigmatism axis of a toric intraocular lens L4 agree with each other. In FIG. 5, the astigmatism cornea lens L3 is formed of a biconvex lens. However, the astigmatism cornea lens L3 may be a meniscus lens or a biconcave lens.

In the schematic eye illustrated in FIG. 5, the toric intraocular lens L4 is arranged in water by estimating the case where the toric intraocular lens L4 is disposed inside the eye. However, the schematic eye may be configured such that the toric intraocular lens L4 is arranged in air provided that a cornea lens having astigmatism can be properly designed and manufactured. As indexes to be observed with respect to the schematic eye illustrated in FIG. 5, Landolt rings described on a visual acuity chart having a length of 3 m are used. An index to be imaged is an optotype of 1.0 vision. A filter which allows light of 546 nm to pass therethrough is mounted on a halogen lump which forms a light source, and light beam which passes through the filter is irradiated to the visual acuity chart from a back side of the visual acuity chart. A camera 10 can move back-and-forth in a direction with respect to an optical axis of the toric intraocular lens L4 for focusing. As illustrated in FIG. 5, the toric intraocular lens L4 is positioned inside an approximately rectangular parallelepiped casing 100 where both surfaces of the casing 100 are formed of planar glasses 101, 101. As described previously, the inside of the casing 100 is filled with water, and a diaphragm S is mounted on an astigmatism cornea lens L3 side of the toric intraocular lens L4.

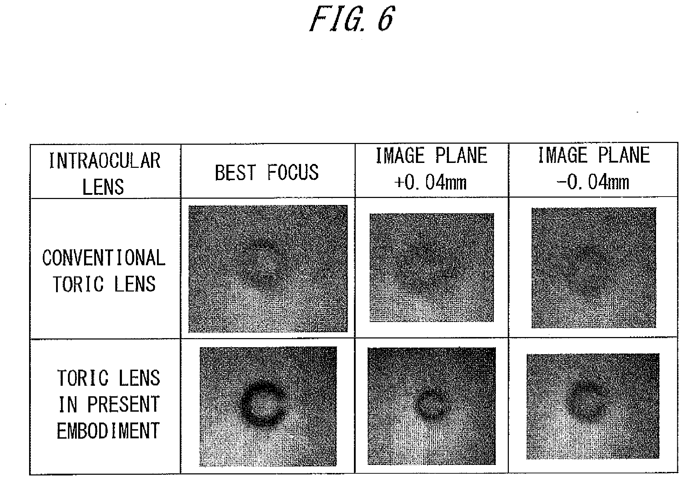

FIG. 6 illustrates a result obtained by imaging Landolt rings which are indexes by a camera when the schematic eye illustrated in FIG. 5 is used. As illustrated in FIG. 6, in the same manner as the simulation result illustrated in FIG. 4, in the toric intraocular lens L4 of this embodiment, Landolt rings are clearly imaged in best focusing. Further, also with respect to images which are intentionally defocused by moving the camera 10 in an optical axis direction of the toric intraocular lens L4, blurring occurs in rotation symmetry. Accordingly, it is understood that astigmatism is favorably reduced. On the other hand, in the conventional toric intraocular lens, although Landolt rings are recognized in best focusing, images are remarkably degraded when the images are intentionally defocused. Accordingly, it is understood that astigmatism is not sufficiently reduced.

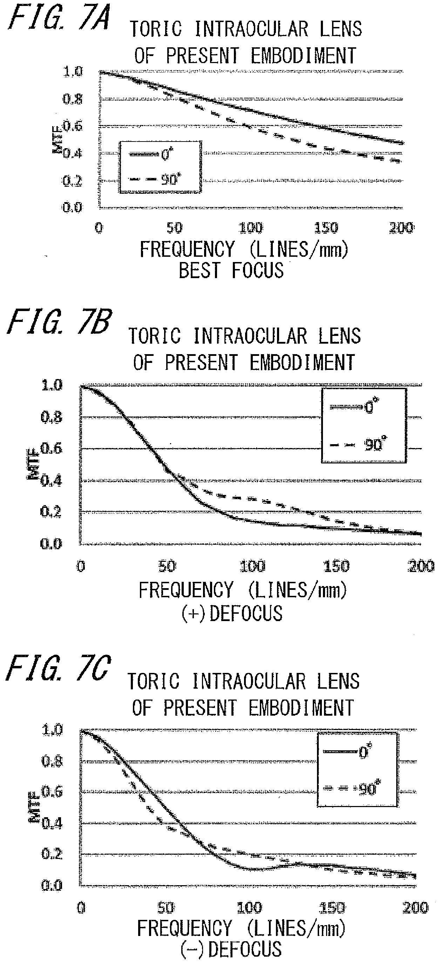

FIGS 7A to 7C illustrate a result obtained by measuring MTF (Modulation Transfer Function) of the toric intraocular lens of this embodiment using the configuration of the schematic eye illustrated in FIG. 6. In the drawings, spatial frequency indicative of a distance of stripes of a stripe pattern used as an object to be imaged is taken on an axis of abscissas, and a value of MTF of an image of the stripe pattern imaged on a light receiving surface of a camera by the toric intraocular lens is illustrated on an axis of ordinates. A solid line indicates numerical values in a sagittal (radiation) direction of the toric intraocular lens (0 degree direction in this case), and a broken line indicates numerical values in a meridional (concentric) direction of the toric intraocular lens (90 degree direction in this case) . As illustrated in FIGS. 7A to 7C, in the case of the schematic eye into which the toric intraocular lens of this embodiment is inserted, it is understood that MTF exhibits favorable values both in the 0 degree direction and in the 90 degree direction in best focusing. Further, even in defocusing, although values of MTF are lowered, MTF exhibits substantially the same change both in the 0 degree direction and in the 90 degree direction and hence, it is understood that astigmatism substantially has not occurred.

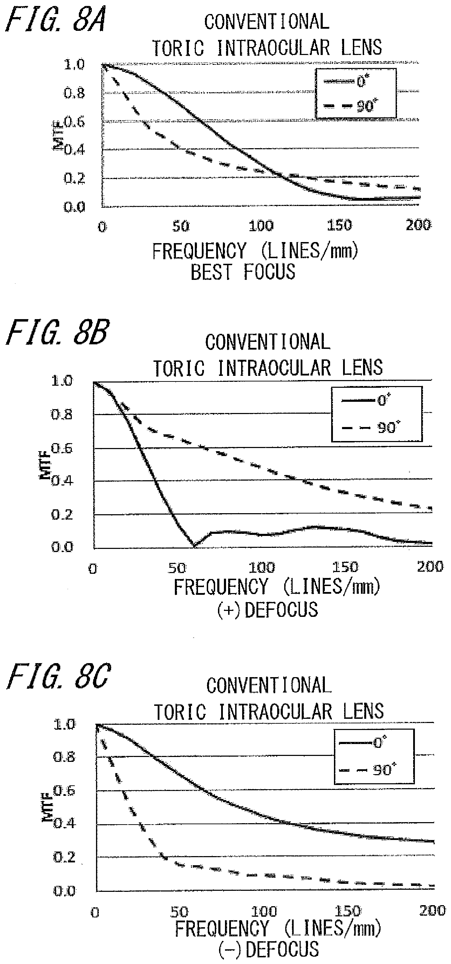

Next, FIGS. 8A to 8C illustrate a result obtained by measuring MTF of a conventional toric intraocular lens using the configuration of the schematic eye illustrated in FIG. 6. As illustrated in FIGS. 8A to 8C, in the conventional toric intraocular lens, MTF at spatial frequency of 100 pieces/mm is 0.2 or more in best focusing and hence, it is considered that the schematic eye can see the vision of 1.0. However, as can be understood from FIGS. 7A to 7C, MTF exhibits low values compared to the toric intraocular lens of this embodiment. Further, as illustrated in FIGS. 8A to 8C, in the conventional toric intraocular lens, MTF exhibits different changes in the 0 degree direction and in the 90 degree direction even at the time of defocusing. Accordingly, it is safe to say that astigmatism still remains in the conventional toric intraocular lens. As described above, with the use of the toric intraocular lens which is designed in the above-mentioned manner by taking into account not only amounts of astigmatism at a center portion of the lens but also amounts of astigmatism at a peripheral portion of the lens in accordance with this embodiment, the correction of astigmatism can be performed more favorably compared to the prior art.

(Embodiment 4)

Next, Embodiment 4, not part of the invention, is described. In the embodiment 4, a toric intraocular lens is designed in accordance with the following specification.

<Design specification>

diameter of incident beam: $\phi$6 mm
refractivity in X direction (under water): +21.5 D (paraxial), +22.0 D (periphery)
refractivity in Y direction (under water) : +18.5 D (paraxial), +18.0 D (periphery)
spherical aberration Z{4,0}RMS: 0.1$\lambda$ or less lens type: biconvex lens (R1 surface: spherical surface, R2 surface: toric surface), wherein |R1|<|R2|
lens material: PMMA
lens central thickness: 0.8 mm
refractive index of water: 1.333 ($\lambda$=546 nm)
wave length of optical source: 546 nm

In the previously described formula (11), assuming x as rcosθ (x=rcosθ), and y as rsinθ (y=rsinθ), the following formula (37) is obtained.

$$Z(x,y) = \frac{c_x x^2 + c_y y^2}{1 + \sqrt{1 - (1+k_x)c_x^2 x^2 - (1+k_y)c_y^2 y^2}}$$

$$(37)$$

This formula expresses a biconic surface.
Hereinafter, a comparison is made with respect to Zernike aberration between the case where a toric intraocular lens is designed using the formula (37) and the case where a toric intraocular lens is designed using the formula (12).
Lens data when a toric intraocular lens is designed using the formula (37) are described in the following Table 7. In Table 7, Ry is a radius of curvature in the y direction, t is a thickness, n is a refractive index, D is a radius, ky is a conic constant in the y direction, Rx is a radius of curvature in the x direction, and kx is a conic constant in the x direction.

[Table 7]

| No | SURFACE | Ry(mm) | t(mm) | n (546nm) | D(mm) | ky | Rx | kx |
|---|---|---|---|---|---|---|---|---|
| 0 | OBJECT SURFACE | inf | inf | 1.336 | 0 | - | - | - |
| 1 | DIAPHRAGM SURFACE | inf | 0 | 1.336 | 3.00 (DIAPHRAGM RADIUS) | - | - | - |
| 2 | R1 SURFACE (SPHERICAL) | 10.8319 | 0.8 | 1.4938 (PMMA) | 3 | - | - | - |
| 3 | R2 SURFACE (TORIC) | -43.6503 | 5 | 1.336 | 3 | -191.184 | -23.967 | -2.337 |
| 4 | IMAGINARY PLANE | inf | 66.48 | 1.336 | 2.78 | - | - | - |
| 5 | IMAGE PLANE | inf | 0 | 1.336 | 0.08 | - | - | - |

Lens data when a toric intraocular lens is designed using the formula (12) are described in the following Table 8.

[Table 8]

| No | SURFACE | R(mm) | t(mm) | n(546nm) | D(mm) | k | A |
|---|---|---|---|---|---|---|---|
| 0 | OBJECT SURFACE | inf | inf | 1.336 | 0 | - | - |
| 1 | DIAPHRAGM SURFACE | inf | 0 | 1.336 | 3.00 (DIAPHRAGM RADIUS) | - | - |
| 2 | R1 SURFACE (SPHERICAL) | 16.0177 | 0.8 | 1.4938 (PMMA) | 3 | - | |
| 3 | R2 SURFACE (TORIC) | -28.5387 | 5 | 1.336 | 3 | - | See TABLE 9 |
| 4 | IMAGINARY PLANE | inf | 66.67 | 1.336 | 2.78 | - | - |
| 5 | IMAGE PLANE | inf | 0 | 1.336 | 0.08 | - | - |

Further, in the formula (12), coefficients are set in accordance with the following Table 9.

[Table 9]

| $a_{2x}$ | $a_{2y}$ | $a_{4x}$ | $a_{2x2y}$ | $a_{4y}$ |
|---|---|---|---|---|
| -1.8313E-02 | -8.9336E-03 | -1.9690E-05 | 1.1230E-04 | 1.3380E-04 |

Under the above-mentioned conditions, Zernike aberrations of the respective designed lenses are described in Table 10. Zernike aberrations are expressed in terms of RMS (Root Mean Square) value (unit: $\lambda$). The order of aberration is set in accordance with Zernike Standard Order.

[Table 10]

| No | FORMULA(12) | FORMULA(37) | ABERRATION |
|---|---|---|---|
| 1 | 0.004 | 0.151 | PISTON |
| 2 | 0 | 0 | SHIFT |
| 3 | 0 | 0 | |
| 4 | <-1E-03 | <-1E-03 | DEFOCUS |
| 5 | 0 | 0 | ASTIGMA TISM |
| 6 | 5.629 | 5.781 | |
| 7 | 0 | 0 | COMA ABERRATION |
| 8 | 0 | 0 | |
| 9 | 0 | 0 | TREFOIL |
| 10 | 0 | 0 | |
| 11 | -0.002 | -0.052 | SPHERICAL ABERRATION |
| 12 | 0.159 | 0.166 | 4TH-ORDER ASTIGMATISM |
| 13 | 0 | 0 | |
| 14 | 0.0003 | 0.2350 | TETRA FOIL |
| 15 | 0 | 0 | |
| 16 | -0.000 | 0.012 | 6TH-ORDER SPHERICAL ABERRATION |

In the above-mentioned calculation, defocusing is set to $10^{-3}$ or less. To compare the aberration of a lens designed using the formula (12) according to the present invention and the aberration of a lens designed using a biconic surface expressed by the formula (37), as illustrated in Table 10, there is a large difference in the aberration in No. 14 (tetra foil aberration). That is, in the lens according to the present invention, aberration can be reduced compared to the lens designed using a biconic surface. Further, the formula according to the present invention requires a shorter converging time which is necessary at the time of designing compared to the conventional formula so that the designing can be performed efficiently. This phenomenon becomes more apparent at the time of designing an ophthalmic lens having large refractivity difference. Further, in manufacturing a lens by a molding method, although there may be a case where the lens has R1 in common with a rotation symmetrical lens, a toric lens can be designed as desired by designing the lens using the formula (12) even in such a case.

The reason is as follows. In the above-mentioned designing using a biconic surface, there are only four parameters Rx, Ry, kx and ky and hence, only the shape in the X direction and the shape in the Y directions can be defined. Accordingly, aberration in the X-Y direction, that is, an arbitrary direction between the X direction and the Y direction cannot be suppressed. On the other hand, in the designing using the formula (12) according to the present invention, the formula (12) has terms such as $X^2Y^2$ which include a variable X and a variable Y, for example, and hence, a surface shape of a lens can be defined also with respect to a direction between the X direction and the Y direction. As a result, undesired aberrations can be eliminated. Further, the aberration referred to as tetra foil aberration is the aberration which has a functional type expressed by $\cos4\theta$ ($\sin4\theta$), while the formula (12) includes a 4$^{th}$ order term when $n\geq2$ as described later, that is, the formula (12) includes a function of $\cos4\theta$ type. Accordingly, aberration can be efficiently eliminated by independently adding a toric surface to a lens by changing parameters.

Recently, along with the development of ophthalmic measuring instruments, it is possible to measure aberrations of an eye in more detail compared to the prior art. Accordingly, the importance of manufacturing a lens which has a function of properly correcting aberrations over the entire lens surface is increasing. According to this embodiment, it is possible to manufacture a lens which has a function of properly correcting aberrations by designing a toric intraocular lens using the formula (11).

(Embodiment 5)

Next, Embodiment 5, not part of the invention, is described. In this embodiment, it is noted that the formula (12) is expressed by the following formula (38).

$$Z = \frac{cr^2}{1+[1-c^2r^2(k+1)]^{1/2}} + \sum_{n=1}^{m}\sum_{j=0}^{n} a_{2jx2(n-j)y} X^{2j} Y^{2(n-j)}$$

$$(38)$$

In the above-mentioned formula (38), m is a natural number, n is a natural number of m or less of m or less, and j is an integer of 0 or more and n or less. In the above-mentioned formula (38), X is a distance from the lens center in the first direction, and Y is a distance from the lens center in the second direction. With the use of this formula (38), a rotation symmetrical lens such as a spherical lens or an aspherical lens can be also designed provided that two conditions described hereinafter are satisfied without being limited to a toric lens. That is, in designing a lens using the formula (12), a comparison between a rotation symmetrical lens and a toric lens can be easily realized by only changing parameters. For example, when a lens formula is changed using an optical soft ZEMAX, one lens data cannot be used in a modified manner, and it is necessary to prepare new lens data. On the other hand, according to this embodiment, a comparison of lenses can be performed using a formula obtained from the formula (12) and hence, a change in parameters can be easily performed using a function of ZEMAX referred to as multi-configuration, for example.

In this embodiment, it is a requisite that the following formulas (39), (40) are satisfied in the formula (38).

$$a_{2nx} = a_{2ny}$$

$$(39)$$

$$a_{2jx2(n-j)y} = \frac{n!}{(n-j)!\,j!} a_{2nx}$$

$$(40)$$

In the above-mentioned formulas, n is a natural number of m or less, and j is an integer of 0 or more and n or less.

As one example, a lens having an aspherical shape expressed by the following formula (41) is considered.

$$Z = \frac{cr^2}{1+[1-c^2 r^2 (k+1)]^{\frac{1}{2}}} + a_2 r^2 + a_4 r^4$$

$$(41)$$

In case of this lens, by setting values indicated in the column "values of coefficients" as respective parameters in the formula (12) as illustrated in following Table 11, it is possible to make a lens surface shape expressed by the formula (12) agree with a lens surface shape expressed by the formula (41).

[Table 11]

| PARAMETERS IN FORMULA (12) | VALUES OF COEFFICIENTS |
|---|---|
| a2x | a2 |
| a2y | a2 |
| a4x | a4 |
| a2x2y | 2a4 |
| a4y | a4 |

It can be regarded that an aspherical lens obtained by setting R, $a_2$ and $a_4$ as R=10.000, $a_2$=0.001 and $a_4$=0.0001 has the same surface shape as a toric lens obtained by setting R, $a_{2x}$, $a_{2y}$, $a_{4x}$, $a_{4y}$ and $a_{2x2y}$ as R=10.000, $a_{2x}$=$a_{2y}$=0.001, $a_{4x}$=$a_{4y}$=0.0001 and $a_{2x2y}$=0.0002.

Further, with respect to the amount of sag in a direction where X is equal to Y (X=Y), as illustrated in the following Table 12, the amount of sag when the lens is designed using the formula (12) and the amount of sag when the lens is designed using the formula (41) agree with each other.

[Table 12]

| IN CASE OF FORMULA (41) | | | IN CASE OF FORMULA (12) | |
|---|---|---|---|---|
| r (mm) | AMOUNT OF SAG (mm) | | $X=Y=\sqrt{r}$ (mm) | AMOUNT OF SAG (mm) |
| O | 0.00000 | | 0 | 0.00000 |
| 0.5 | 0.012764 | | 0.35355 | 0.012764 |
| 1.0 | 0.051226 | | 0.70711 | 0.051226 |
| 1.5 | 0.115896 | | 1.06066 | 0.115896 |
| 2.0 | 0.207641 | | 1.41421 | 0.207641 |
| 2.5 | 0.327698 | | 1.76777 | 0.327698 |
| 3.0 | 0.477708 | | 2.12132 | 0.477708 |

Although the amount of sag in the direction where X is equal to Y (X=Y) is described as one example, it is understood that the amount of sag in the case where the lens is designed using the formula (12) and the case where the lens is designed using the formula (41) are equal also in an arbitrary direction. Accordingly, by setting parameters such that only terms including $X^{2n}$ and $Y^{2n}$ are used in second and succeeding terms of the formula (12) due to formulas (39) and (40), in the 45 degree direction (that is, X=Y), the lens surface becomes substantially equal to a reference surface shape to which a toric surface is not added. Accordingly, the mutual correlation among a plurality of product groups (spherical surface, aspherical surface, toric surface and the like) to which lens designing is applied using the formula (12) and the evaluation of the product groups can be easily performed. The formula (38) may be also used in place of the formula (12) also in other embodiments.

(Embodiment 6)

Next, Embodiment 6, not part of the invention, is described. In this embodiment, a toric surface obtained by adding a definition formula (n: natural number) based on $(X+Y)^{2n-1}$ to the formula (12) is used. In designing a toric intraocular lens, alignment between an astigmatism axis and a toric axis is important. Accordingly, it is also necessary to evaluate the misalignment between the astigmatism axis and the toric axis. Further, an edge thickness of the toric intraocular lens is not constant and changes. However, with the use of general optical software, it is possible to calculate only an edge thickness in the X direction or in the Y direction. Accordingly, it is necessary to perform an operation of rotating an optical system of a lens in the software or calculate the edge thickness based on the difference between the edge thickness and the central thickness of the lens by calculating the amount of sag of a toric surface.

In this embodiment, the lens can be rotated as desired by performing the conversion of parameters as described below. By performing such an operation, it is possible to set meridians (diameters) having desired angles on the X axis and Y axis in the software and hence, an amount of calculation at the time of designing the lens can be suppressed. For example, assuming the respective differences between coefficients of $X^2$ and coefficients of $Y^2$ in the formula (12) as coefficients of XY, the lens can be rotated by 45° (or -45°).

The lens can be rotated as desired by converting variables using the following formula (42).

$$\begin{pmatrix} X' \\ Y' \\ Z' \end{pmatrix} = \begin{pmatrix} \cos\theta & -\sin\theta & 0 \\ \sin\theta & \cos\theta & 0 \\ 0 & 0 & 1 \end{pmatrix} \begin{pmatrix} X \\ Y \\ Z \end{pmatrix} = \begin{pmatrix} X\cos\theta - Y\sin\theta \\ X\sin\theta + Y\cos\theta \\ Z \end{pmatrix} \qquad (42)$$

In the formula (42), $\theta$ is a rotational angle, X', Y' and Z' are coefficients and variables after conversion, and X, Y and Z are variables before rotation.

As one example, a toric surface expressed by the following formula (43) which is obtained from the formula (12) is considered.

$$Z = \frac{cr^2}{1 + [1 - c^2 r^2 (k+1)]^{\frac{1}{2}}} + a_{2x}X^2 + a_{2y}Y^2 \qquad (43)$$

In the case where the toric surface is rotated only by $\theta$, the second term and the third term of the formula (43) are converted as described in the following formula (44). The first term in the formula (43) expresses a rotation symmetrical lens shape and hence, the description of the conversion is omitted.

$$a_{2x}X^2 + a_{2y}Y^2 = a_{2x}(X\cos\theta - Y\sin\theta)^2 + a_{2y}(X\sin\theta + Y\cos\theta)^2$$

$$= (a_{2x}\cos^2\theta + a_{2y}\sin^2\theta)X^2 + (a_{2x}\sin^2\theta + a_{2y}\cos^2\theta)Y^2 + 2(-a_{2x} + a_{2y})\sin\theta\cos\theta XY$$

$$(44)$$

In the above-mentioned conversion, variables having degrees up to 2 are subjected to the conversion. However, also when a lens has a lens shape expressed by a formula having coefficients of higher degrees, the lens can be rotated at a desired angle by performing the calculation in the same manner as described above.

As one example, in the case where a lens having a toric surface where R, k, $a_{2x}$ and $a_{2y}$ are set as R=10.000, k=0, $a_{2x}$=0.001 and $a_{2y}$=-0.001 is rotated by 30 degrees, the toric surface after the rotation is expressed as a surface where R, k, $a_{2x}$, $a_{xy}$, $a_{2x2y}$ are set as R=10.000, k=0, $a_{2x}$=0.0005, $a_{xy}$=-0.0017321 and $a_{2x2y}$=-0.0005. When such a lens having the toric surface is rotated by 15 degrees in the same manner, the toric surface after the rotation is expressed as a surface where R, k, $a_{2x}$, $a_{xy}$, $a_{2x2y}$ are set as R=10.000, k=0, $a_{2x}$=0.00086603, $a_{xy}$=0.001 and $a_{2x2y}$=-0.00086603.

In case of a toric intraocular lens, usually, an edge thickness changes at a period of 180° about an optical axis. In a conventional toric intraocular lens, for example, as described in JP-T-2011-519682, when an edge thickness changes in a sinusoidal manner about the optical axis, due to a characteristic of a sinusoidal function (sin function), a function which is displaced by 90° agree with a cos function and hence, a change in edge thickness in the vicinity of a steep meridian and a change in edge thickness in the vicinity of a flat meridian are substantially equal. In this manner, an edge thickness of a sinusoidal toric intraocular lens changes in accordance with sin2θ (or cos2θ), while an edge thickness of a toric intraocular lens designed by the formula (12) changes in accordance with cos2θ+cos4θ since the formula (12) includes terms of $X^4$, $Y^4$ and $X^2Y^2$. Accordingly, although the period of change in edge thickness is 180°, the change in edge thickness in the vicinity of the steep meridian and the change in edge thickness in the vicinity of the flat meridian differ from each other.

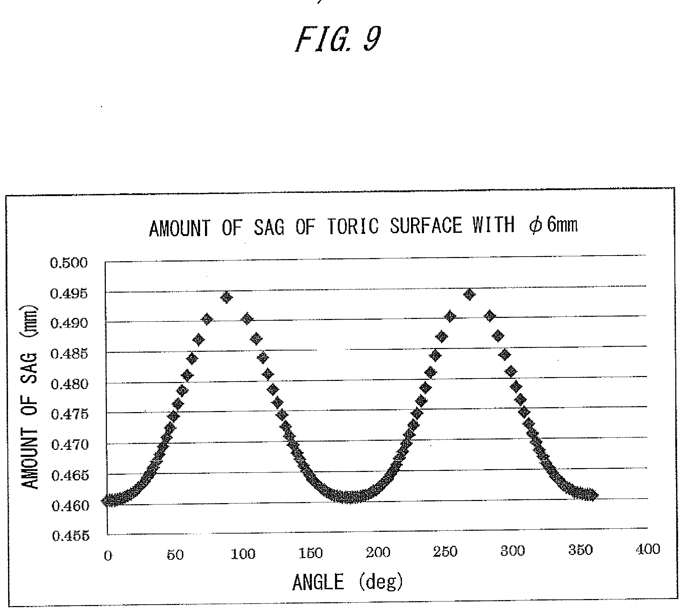

As one example, FIG. 9 illustrates a result obtained by plotting the amount of sag at a point of $\phi6$ mm with respect to a toric surface obtained by setting R, k, $a_{2x}$ and $a_{4x}$ as R=10.000, k=0, $a_{2x}$=0.001 and $a_{4x}$=0.0003 in the formula (12). As illustrated in FIG. 9, a change in the amount of sag is steep in the vicinity of 90° and 270°, while the change in the amount of sag is gentle at 0°, 180° and 360°. With respect to a function which changes in this manner, unlike a function which changes in a sinusoidal manner in general, the function within a range of from 0° to 270° is not formed in rotation symmetry with respect to 135° which is the middle of the range. Being small in amount of change in edge thickness means that a lens shape approaches a rotation symmetrical shape. That is, a change in edge thickness is small in the vicinity of 0°, 180° and 360° so that it is possible to design a toric intraocular lens which exhibits behavior close to behavior of a rotation symmetrical lens. Accordingly, in this embodiment, an amount of change in edge thickness in the vicinity of the flat meridian or the steep meridian of the lens can be made small. Therefore, it is possible to design a toric intraocular lens which can be easily installed in an inserting instrument in the same manner as the rotation symmetrical lens at the time of inserting the lens into an eye in an folded manner, and can be transported in a stable manner.

(Embodiment 7)

Next, Embodiment 7, according to the invention, is described. In general, in the manufacture of an optical part, a step for checking the part is provided. An optical surface of a general rotation symmetry system is given by the following formula (45).

$$Z = \frac{cr^2}{1+[1-c^2r^2(k+1)]^{1/2}} + a_2 r^2 + a_4 r^4 + \cdots$$

$$(45)$$

The optical surface given by the formula (45) is in rotation symmetry with respect to an optical axis. Therefore, the same evaluation result is obtained even when the optical surface is evaluated in any diametrical direction. However, in case of evaluating a rotation asymmetrical optical surface such as an optical surface of a toric lens, with respect to a conventional toric surface, the evaluation in directions other than an axial direction (X=0 or Y=0) was extremely difficult. On the other hand, by defining a surface shape using the formula (12) of the present invention, a cross-sectional shape in an arbitrary direction can be easily estimated and expressed compared to a conventional toric surface shape.

In this embodiment, an expression formula of a cross-sectional shape of an optical surface of a lens at an arbitrary direction (angle θ) is induced from the formula (12). In this embodiment, as one example, the case is considered where the maximum degree in the formula (12) is 4. Assuming x and y as x=rcosθ and y=rsinθ in the formula (12), the formula (46) is obtained by converting the formula (12) as described below.

$$Z = \frac{cr^2}{1+[1-c^2r^2(k+1)]^{1/2}} + a_{2x}X^2 + a_{2y}Y^2 + a_{4x}X^4 + a_{2x2y}X^2Y^2 + a_{4y}Y^4$$

$$= \frac{cr^2}{1+[1-c^2r^2(k+1)]^{1/2}} + (a_{2x}\cos^2\theta + a_{2y}\sin^2\theta)r^2 + (a_{4x}\cos^4\theta + a_{2x2y}\cos^2\theta\sin^2\theta + a_{4y}\sin^4\theta)r^4$$

$$= \frac{cr^2}{1+[1-c^2r^2(k+1)]^{1/2}} + A(\theta)r^2 + B(\theta)r^4$$

$$(46)$$

In the formula (46), A(θ) and B(θ) are expressed by the following formulas (47).

$$A(\theta) = a_{2x} \cos^2 \theta + a_{2y} \sin^2 \theta \;,\; B(\theta) = a_{4x} \cos^4 \theta + a_{2x2y} \cos^2 \theta \sin^2 \theta + a_{4y} \sin^4 \theta$$

$$(47)$$

Further, in the formula (46), A(θ) and B(θ) are expressed by the following formulas (48).

$$A(\theta) = \frac{1}{2}[a_{2x} + a_{2y} + (a_{2x} - a_{2y})\cos 2\theta \;,$$

$$B(\theta) = \frac{1}{8}[(3a_{4x} + a_{2x2y} + 3a_{4y}) + 4(a_{4x} - a_{4y})\cos 2\theta + (a_{4x} - a_{2x2y} + a_{4y})\cos 4\theta]$$

$$(48)$$

As can be understood from the formula (46), with the use of the formula (12), a cross-sectional shape of a lens surface in an arbitrary direction (arbitrary θ) can be expressed using a general optical surface definition formula. The realization of expressing a cross-sectional shape of a lens surface using a general optical surface definition formula is extremely convenient for evaluating a lens. This is because, for example, with the use of a software installed in a commercially available non-contact three dimensional size measuring device NH-3SP made by Mitaka Kohki Ltd. , a measured cross-sectional shape can be modified to a shape of the above-mentioned formula (45) by fitting. Further, a comparison between measured values and designed values and optical simulation within a desired cross section of an actually manufactured lens can be easily performed.

On the other hand, in the case of a conventional toric optical surface (in the case of the optical surfaces expressed by the formula (11) or the formula (37)), although a cross-sectional shape agrees with the first term of the general formula (45) in the case where X or Y is set to 0 ((X=0 or Y=0) (θ=0° or θ=90°)), it is difficult to express the cross-sectional shape in the form of the general formula (45) when neither X nor Y are 0 (X≠0 and Y≠0), that is, it is extremely difficult to obtain k, c and a which are applicable to the formula (45).

Accordingly, in the evaluation of a cross-sectional shape other than a cross-sectional shape on an axis of the lens manufactured using an expression formula of a lens used conventionally, since the cross-sectional shape of the lens is extremely complicated, it is necessary to prepare a special shape evaluation software. Further, with respect to a toric surface expressed by the formula (7) to the formula (10), except for the case where X or Y is 0 (X=0 or Y=0), a shape of the lens changes depending on a working method. Therefore, the adjustment of the shape is extremely difficult.

Further, in applying optical calculation to a lens surface, it is necessary to calculate the inclination of a lens surface at an arbitrary position, and the differentiation of a function becomes necessary for calculating the inclination. The definition formula of the present invention expressed by the formula (12) or the formula (38) does not include other functions (for example, triangular function) in a rout and hence, the differential calculation can be easily performed. Further, also in confirming that an operation of a lens inserting instrument is not obstructed even when an intraocular lens manufactured using the definition formula of the present invention is installed into the lens inserting instrument, it is possible to calculate a cross-sectional area of the lens by easily applying integral calculation to the definition formula.

A desired lens surface can be combined with a surface defined by the formula (12) or the formula (38) of the present invention. For example, by properly setting spherical aberration by combining proper aspherical surfaces, the degradation of an image may be alleviated even when an axis of a toric lens is displaced from an astigmatism axis. Such setting of the spherical aberration can be performed on a surface defined by the definition formula of the present invention. For example, such setting of the spherical aberration can be realized in a portion of the first term in the formula (12) or the formula (38). Alternatively, such setting of the spherical aberration may be realized using parameters in the second and subsequent terms in the formula (12) or the formula (38) .

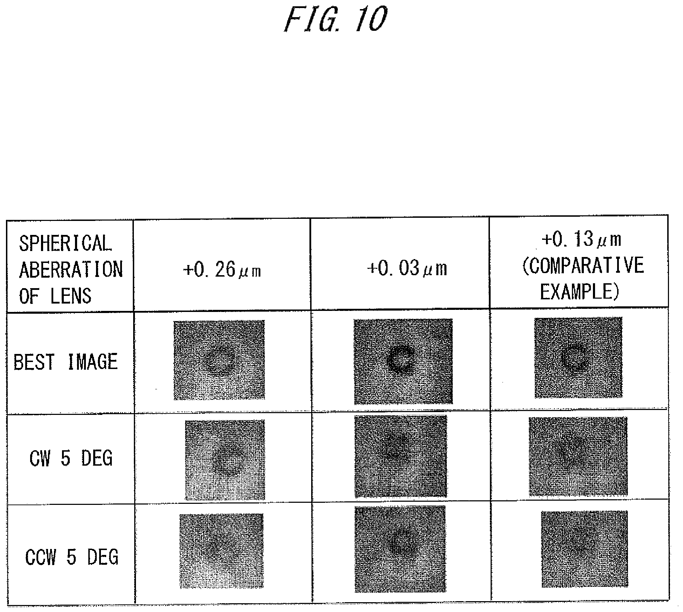

Here, an evaluation result with respect to axis misalignment in a toric lens combined with an aspherical surface which controls spherical aberration is described. The evaluation is performed using a schematic eye illustrated in FIG. 5. FIG. 10 illustrates a result obtained by imaging Landolt rings which are indexes by a camera when the axis misalignment occurs in using the schematic eye illustrated in FIG. 5. FIG. 10 illustrates a state of the Landolt rings imaged by rotating the toric lens by ±5° from a state where an astigmatism axis of a cornea lens and an axis of a toric IOL (Intraocular Lens) are made to agree with each other when a spherical aberration amount is changed as the schematic eye inserted into an IOL. Here, testing conditions in FIG. 10 are as follows.
cornea lens: PMMA
cornea refractivity: flat meridians 40.4 D, steep meridians 42.4 D
cornea spherical aberration: +0.28 $\mu$m (@ $\phi$6 mm)
diaphragm diameter: $\phi$5.2 mm(@ IOL front surface)
IOL: cylindrical refractivity 3.0 D, equivalent
spherical power 20 D

As illustrated in FIG. 10, when spherical aberration which is obtained by combining spherical aberration of an IOL and spherical aberration of a cornea of an eyeball into which the IOL is inserted is approximately 0 (+0.03 $\mu$m in FIG. 10), that is, when the spherical aberration of the IOL is approximately -0.28 $\mu$m, a Landolt ring can be clearly observed in a state where an astigmatism axis of the cornea lens and an axis of the IOL agree with each other. However, when the lens is rotated so that the astigmatism axis of the cornea lens and the axis of the IOL are displaced from each other, an image of the Landolt ring is remarkably degraded and hence, the Landolt ring cannot be recognized with a naked eye. On the other hand, when spherical aberration which is obtained by combining spherical aberration of the IOL and spherical aberration of the cornea of the eyeball into which the IOL is inserted falls within a range of 0.2 $\mu$m to 0.3 $\mu$m (+0.26 $\mu$m in FIG. 10), that is, when the spherical aberration of the IOL falls within a range of -0.08 $\mu$m to +0.02 $\mu$m, a Landolt ring can be observed in a state where an astigmatism axis of the cornea lens and an axis of the IOL agree with each other and, at the same time, the Landolt ring can be recognized even when the lens is rotated so that the astigmatism axis of the cornea lens and the axis of the IOL are displaced from each other. In FIG. 10, in the case of spherical aberration of an IOL (+0.13 $\mu$m) of the comparison example, an image which is at an approximately intermediate position between the above-mentioned two kinds of images is formed in a state where an astigmatism axis of the cornea lens and an axis of the IOL agree with each other. However, when the lens is rotated so that the astigmatism axis of the cornea lens and the axis of the IOL are displaced from each other, an image where Landolt rings overlap longitudinally or laterally is formed. Accordingly, it is difficult to regard this state as a state where a Landolt ring can be recognized with a naked eye.

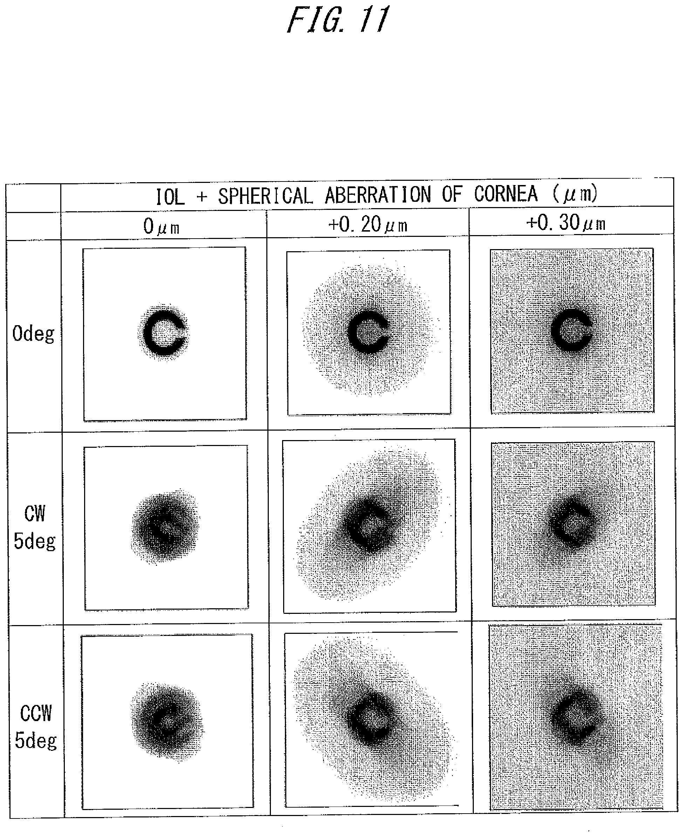

Next, a result obtained by performing an optical simulation of a retina image in the above-mentioned schematic eye is illustrated in FIG. 11. As illustrated in FIG. 11, when spherical aberration which is obtained by combining spherical aberration of the IOL and spherical aberration of the cornea of the eyeball into which the IOL is inserted is 0, that is, when the spherical aberration of the IOL is approximately -0.28 $\mu$m, a Landolt ring can be clearly observed in a state where the astigmatism axis of the cornea lens and the axis of the IOL agree with each other. However, when the lens is rotated so that the astigmatism axis of the cornea lens and the axis of the IOL are displaced from each other, an image of the Landolt ring is remarkably degraded and the Landolt ring cannot be recognized with a naked eye. This result agrees with the evaluation result of the actual device illustrated in FIG. 10 and hence, it is considered that the accuracy of the optical simulation is proved.

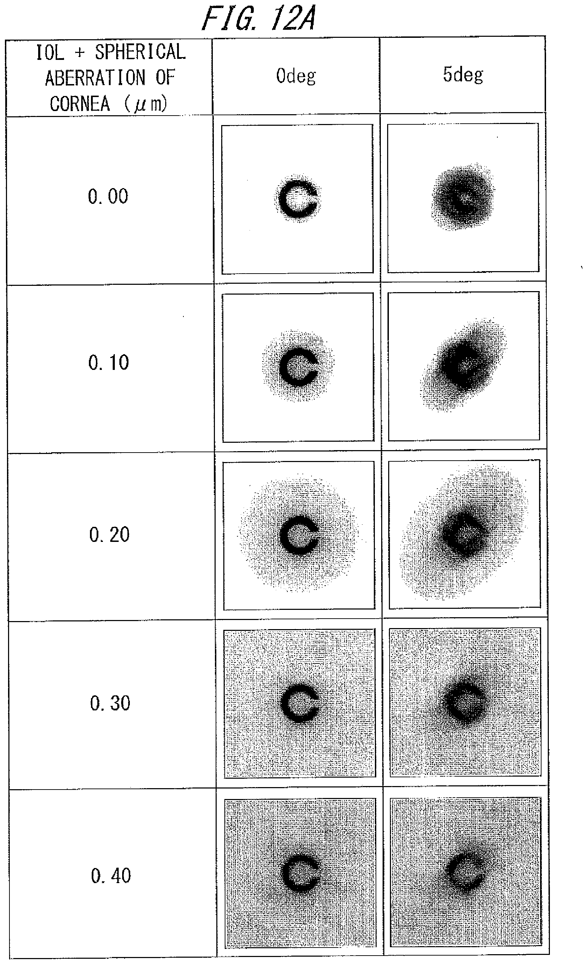

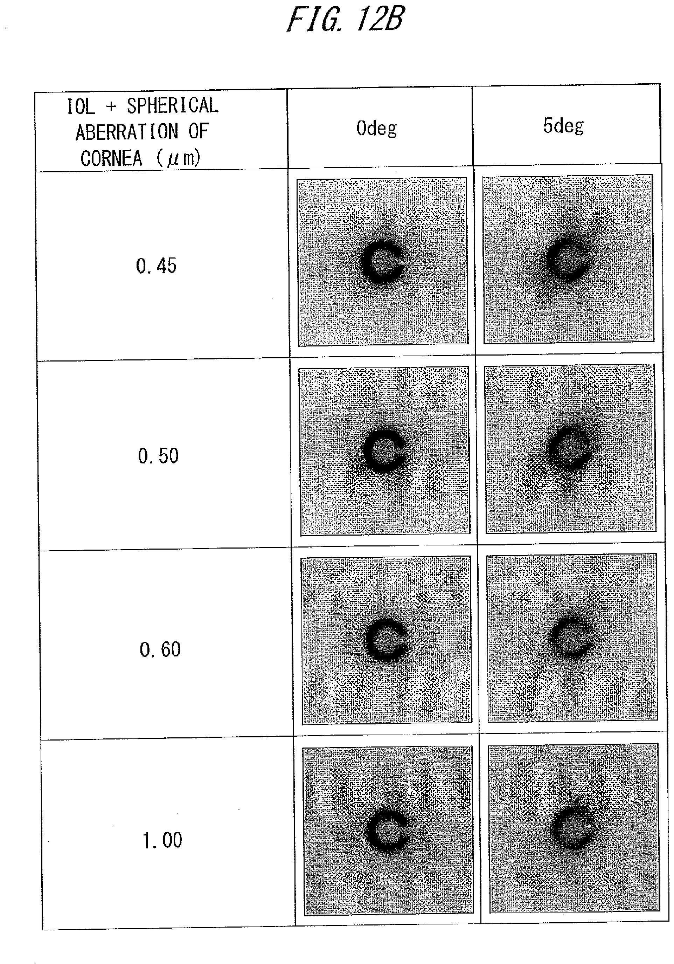

To further study a result obtained by performing an optical simulation illustrated in FIG. 12A and FIG. 12B, it is understood that when spherical aberration which is obtained by combining spherical aberration of the IOL and spherical aberration of the cornea of the eyeball into which the IOL is inserted falls within a range of 0.2 $\mu$m to 0.3 $\mu$m, that is, when the spherical aberration of the IOL falls within a range of -0.08 $\mu$m to +0.02 $\mu$m, a Landolt ring can be observed in a state where an astigmatism axis of the cornea lens and an axis of the IOL agree with each other and, at the same time, the Landolt ring can be recognized even when the lens is rotated so that the astigmatism axis of the cornea lens and the axis of the IOL are displaced from each other. It is also understood that when spherical aberration which is obtained by combining spherical aberration of the IOL and spherical aberration of the cornea of the eyeball into which the IOL is inserted is larger than 0.5 $\mu$m, that is, when the spherical aberration of the IOL is larger than +0.22 $\mu$m, although the degradation between Landolt rings caused by the axis misalignment is small, contrast in a state where the astigmatism axis of the cornea lens and the axis of the IOL agree with each other is lowered thus giving rise to a concern that quality of an image is lowered as a whole.

The intraocular lens of this disclosure can be manufactured by a molding method or a cutting working method. However, it is desirable to perform forming of a toric surface using a lathe which can move a working tool in an optical axis direction in synchronism with a rotational speed.

[Reference Signs List]

L4    toric intraocular lens

**Claims**

1. An ophthalmic lens having a cross-sectional shape in an arbitrary meridian direction on a lens surface of the ophthalmic lens, **characterized in that** the ophthalmic lens is a toric lens and the cross-sectional shape is expressed by the following formula (1),

$$Z = \frac{cr^2}{1 + [1 - c^2 r^2 (k+1)]^{\frac{1}{2}}} + A(\theta)r^2 + B(\theta)r^4$$

$$(1)$$

wherein c is a paraxial curvature of the ophthalmic lens, r is a distance from a lens center of the ophthalmic lens, k is a conic constant of a surface which is in rotation symmetry with respect to an optical axis of the lens in the ophthalmic lens, c, r and k are used in common in the meridian direction on the lens surface, and A ($\theta$) and B ($\theta$) are expressed by the following formulas (2) and (3),

$$A(\theta) = a_{2x} \cos^2 \theta + a_{2y} \sin^2 \theta$$

$$(2)$$

$$B(\theta) = a_{4x} \cos^4 \theta + a_{2x2y} \cos^2 \theta \sin^2 \theta + a_{4y} \sin^4 \theta$$

$$(3)$$

where $\theta$ is the angle in the meridian direction about the optical axis of the lens and $a_{2x}$, $a_{2y}$, $a_{4x}$, $a_{2x2y}$, $a_{4y}$ are settable parameters.

2. The ophthalmic lens according to claim 1, wherein A($\theta$) in the formula (1) is a function having a period of 180°, and B($\theta$) is a sum of the function having a period of 180° and the function having a period of 90°.

3. A method for designing an ophthalmic lens, according to claim 1, the method comprising obtaining the cross-sectional shape of the ophthalmic lens by formula (1), where A($\theta$) and B($\theta$) are obtained by formulas (2) and (3).

4. A method according to claim 3, wherein A($\theta$) in the formula (1) is a function having a period of 180°, and B($\theta$) is a sum of the function having a period of 180° and the function having a period of 90°.

**Patentansprüche**

1. Ophthalmische Linse mit einer Querschnittsform in einer willkürlichen Meridianrichtung auf einer Linsenoberfläche der ophthalmischen Linse, **dadurch gekennzeichnet, dass** die ophthalmische Linse eine torische Linse ist und die Querschnittsform durch die folgende Formel (1) beschrieben wird,

$$Z = \frac{cr^2}{1 + [1 - c^2 r^2 (k+1)]^{1/2}} + A(\theta)r^2 + B(\theta)r^4 \qquad (1)$$

wobei c eine paraxiale Krümmung der ophthalmischen Linse ist, r ein Abstand von einer Linsenmitte der ophthalmischen Linse ist, k eine Konuskonstante einer Fläche ist, die in Rotationssymmetrie in Bezug auf eine optische Achse der Linse in der ophthalmischen Linse ist, c, r und k werden gemeinsam in der Meridianrichtung auf der Linsenoberfläche beschrieben, und A($\theta$) und B($\theta$) werden durch die folgenden Formeln (2) und (3) beschrieben,

$$A(\theta) = a_{2x} cos^2\theta + a_{2y}\sin^2\theta \qquad (2)$$

$$B(\theta) = a_{4x} cos^4\theta + a_{2x2y} cos^2\theta \sin^2\theta + a_{4y}\sin^4\theta \qquad (3)$$

wobei $\theta$ der Winkel in Meridianrichtung um die optische Achse der Linse und $a_{2x}$, $a_{2y}$, $a_{4x}$, $a_{2x2y}$, $a_{4y}$ einstellbare Parameter sind.

2. Ophthalmische Linse nach Anspruch 1, wobei A($\theta$) in der Formel (1) eine Funktion ist, die eine Periode von 180° aufweist, und B($\theta$) eine Summe der Funktion, die eine Periode von 180° aufweist, und der Funktion mit einer Periode von 90° ist.

3. Verfahren zum Entwerfen einer ophthalmischen Linse nach Anspruch 1, wobei das Verfahren ein Erhalten der Querschnittsform der ophthalmischen Linse durch Formel (1) umfasst, wobei A($\theta$) und B($\theta$) durch die Formeln (2) und (3) erhalten werden.

4. Verfahren nach Anspruch 3, wobei A($\theta$) in der Formel (1) eine Funktion mit einer Periode von 180° ist, und B($\theta$) eine Summe aus der Funktion mit einer Periode von 180° und der Funktion mit einer Periode von 90° ist.

**Revendications**

1. Lentille ophtalmique ayant une forme en coupe transversale dans une quelconque direction méridienne sur une surface de lentille de la lentille ophtalmique, **caractérisée en ce que** la lentille ophtalmique est une lentille torique et **en ce que** la forme en coupe transversale est exprimée par la formule (1) suivante :

$$Z = \frac{cr^2}{1+[1-c^2r^2(k+1)]^{\frac{1}{2}}} + A(\theta)r^2 + B(\theta)r^4$$

$$(1)$$

dans laquelle c'est une courbe paraxiale de la lentille ophtalmique, r est une distance depuis un centre de lentille de la lentille ophtalmique, k est une constante conique d'une surface qui est dans une symétrie de rotation par rapport à un axe optique de la lentille dans la lentille ophtalmique, c, r et k sont utilisés en commun dans la direction méridienne sur la surface de lentille, et A(θ) et B(θ) sont exprimés par les formules (2) et (3) suivantes,

$$A(\theta) = a_{2x} \cos^2\theta + a_{2y} \sin^2\theta$$

$$(2)$$

$$B(\theta) = a_{4x} \cos^4\theta + a_{2x2y} \cos^2\theta\sin^2\theta + a_{4y} \sin^4\theta$$

$$(3)$$

dans lesquelles θ est l'angle dans la direction méridienne autour de l'axe optique de la lentille et $a_{2x}$, $a_{2y}$, $a_{4x}$, $a_{2x2y}$, $a_{4y}$ sont des paramètres ajustables.

2. Lentille ophtalmique selon la revendication 1, dans laquelle A(θ) dans la formule (1) est une fonction ayant une période de 180°, et B(θ) est une somme de la fonction ayant une période de 180° et de la fonction ayant une période de 90°.

3. Procédé de conception d'une lentille ophtalmique, selon la revendication 1, le procédé comprenant l'obtention de la forme en coupe transversale de la lentille ophtalmique par la formule (1), où A(θ) et B(θ) sont obtenus par les formules (2) et (3).

4. Procédé selon la revendication 3, dans lequel $A(\theta)$ dans la formule (1) est une fonction ayant une période de 180°, et $B(\theta)$ est une somme de la fonction ayant une période de 180° et de la fonction ayant une période de 90°.

## FIG. 1

SPHERICAL ABERRATION

PARAXIAL FOCAL POINT F

FOCAL LENGTH f

DISTANCE FROM REAR SIDE
MAIN POINT TO FOCAL POINT
OF PERIPHERAL LIGHT BEAM f'

## FIG. 2A

L 2    H'

SPHERICAL ABERRATION
IN X-AXIS DIRECTION

PARAXIAL FOCAL POINT
IN X-AXIS DIRECTION Fx

FOCAL POINT OF
PERIPHERAL RAY
IN X-AXIS DIRECTION Fx'

FOCAL LENGTH
IN X-AXIS DIRECTION fx

DISTANCE FROM REAR SIDE
MAIN POINT TO FOCAL POINT
OF PERIPHERAL LIGHT BEAM
IN X-AXIS DIRECTION fx'

## FIG. 2B

L 2    H'

SPHERICAL ABERRATION
IN Y-AXIS DIRECTION

PARAXIAL FOCAL
POINT IN Y-AXIS
DIRECTION Fy

FOCAL POINT OF
PERIPHERAL RAY
IN Y-AXIS
DIRECTION Fy'

DISTANCE FROM REAR SIDE
MAIN POINT TO FOCAL POINT
OF PERIPHERAL LIGHT BEAM
IN Y-AXIS DIRECTION fy'

FOCAL LENGTH
IN Y-AXIS DIRECTION fy

# FIG. 3

# FIG. 4

| INTRAOCULAR LENS | BEST FOCUS | IMAGE PLANE +0.04mm | IMAGE PLANE −0.04mm |
|---|---|---|---|
| CONVENTIONAL TORIC LENS | | | |
| TORIC LENS IN PRESENT EMBODIMENT | | | |

# FIG. 5

# FIG. 6

| INTRAOCULAR LENS | BEST FOCUS | IMAGE PLANE +0. 04mm | IMAGE PLANE −0. 04mm |
|---|---|---|---|
| CONVENTIONAL TORIC LENS | | | |
| TORIC LENS IN PRESENT EMBODIMENT | | | |

## FIG. 7A

TORIC INTRAOCULAR LENS
OF PRESENT EMBODIMENT

FREQUENCY (LINES/mm)
BEST FOCUS

## FIG. 7B

TORIC INTRAOCULAR LENS
OF PRESENT EMBODIMENT

FREQUENCY (LINES/mm)
(+) DEFOCUS

## FIG. 7C

TORIC INTRAOCULAR LENS
OF PRESENT EMBODIMENT

FREQUENCY (LINES/mm)
(−) DEFOCUS

*FIG. 8A*

CONVENTIONAL
TORIC INTRAOCULAR LENS

FREQUENCY (LINES/mm)
BEST FOCUS

*FIG. 8B*

CONVENTIONAL
TORIC INTRAOCULAR LENS

FREQUENCY (LINES/mm)
(+) DEFOCUS

*FIG. 8C*

CONVENTIONAL
TORIC INTRAOCULAR LENS

FREQUENCY (LINES/mm)
(−) DEFOCUS

# FIG. 9

AMOUNT OF SAG OF TORIC SURFACE WITH $\phi$ 6mm

## FIG. 10

| SPHERICAL ABERRATION OF LENS | +0.26 $\mu$m | +0.03 $\mu$m | +0.13 $\mu$m (COMPARATIVE EXAMPLE) |
|---|---|---|---|
| BEST IMAGE | | | |
| CW 5 DEG | | | |
| CCW 5 DEG | | | |

## FIG. 11

| | IOL + SPHERICAL ABERRATION OF CORNEA ($\mu$m) | | |
|---|---|---|---|
| | $0\mu$m | $+0.20\mu$m | $+0.30\mu$m |
| 0deg | | | |
| CW 5deg | | | |
| CCW 5deg | | | |

# FIG. 12A

| IOL + SPHERICAL ABERRATION OF CORNEA (μm) | 0deg | 5deg |
|---|---|---|
| 0.00 | | |
| 0.10 | | |
| 0.20 | | |
| 0.30 | | |
| 0.40 | | |

## FIG. 12B

| IOL + SPHERICAL ABERRATION OF CORNEA (μm) | 0deg | 5deg |
|---|---|---|
| 0.45 | | |
| 0.50 | | |
| 0.60 | | |
| 1.00 | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4945558 B
- JP 2011519682 A
- US 2011205486 A

- WO 0189424 A
- DE 102008003770 A
- JP 2011519682 T